# EUROPEAN PATENT APPLICATION

(11) **EP 2 982 382 A1**
(43) Date of publication of application: **10.02.2016**
(21) Application number: 14179771.2
(22) Date of filing: 04.08.2014
(51) Int. Cl.: A61K 45/06, A61K 31/4174, A61K 31/4178, A61K 31/4196, A61K 31/427, A61P 27/16

(54) **Compounds for preventing ototoxicity**

(71) Applicant: Sensorion, 34000 Montpellier (FR)
(72) Inventor: Gaboyard-Niay, Sophie, 34770 Gigean (FR)
(74) Representative: Icosa

(57) **Abstract**

The present invention relates to a compound for use for preventing ototoxicity in a subject in need thereof. The present invention also relates to a composition, a pharmaceutical composition or a medicament comprising the compound of the invention.

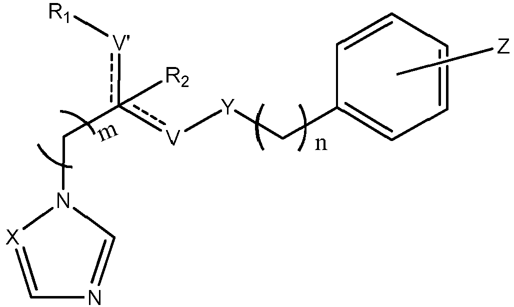

## Description

### FIELD OF INVENTION

The present invention relates to methods for preventing ototoxicity. More specifically, the present invention relates to compounds for preventing ototoxicity, such as, for example, drug-induced ototoxicity.

### BACKGROUND OF INVENTION

There are several causes to ototoxicity: exposure to loud noise, head and neck radiation, but ototoxicity is more frequently caused by drug reactions. Ototoxic drugs include aminoglycoside antibiotics such as gentamicin, platinum-based chemotherapy agents such as cisplatin, excitatory neurotoxins such as glutamate, loop-diuretics, contaminants in food or medicaments or environmental and industrial pollutants. Symptoms of ototoxicity include partial or profound hearing loss, vertigo, tinnitus, and dizziness. If the ototoxic drug is used at high doses and/or for a prolonged time, the impairments can become persistent and irreversible.

Methods for treating ototoxicity were described in the art. For example, WO 2012/112810 and US 2011/0046156 describe the administration of multiparticulate corticosteroids and redox-active therapeutics respectively, for treating or reducing the severity of ototoxicity. However, the two of them only describe the treatment of a symptom of ototoxicity but do not prevent ototoxicity.

Some ototoxicity preventing agents are known, such as cimetidine, thiourea, pantoprazole or sodium thiosulfate. Cimetidine is an approved drug for the treatment of heartburn and peptic ulcers, but also has potential as a clinical antidote against cisplatin-induced nephrotoxicity. According to preclinical data, cimetidine also seems to have potential as a clinical antidote against cisplatin-induced ototoxicity. Unfortunately, this agent can lead to side effects because of the high therapeutically effective dose necessary to observe an effect. Besides WO 03061574 describes thiourea as an ototoxic preventing agent. Moreover, as regards sodium thiosulfate, preliminary results from phase 3 studies seem to show a lower survival of administered patients (Freyer DR, "The effects of sodium thiosulfate (STS) on cisplatin-induced hearing loss: A report from the Children's Oncology Group.", Abstract 10017. 2014 ASCO Annual Meeting).

Therefore, to the Applicant knowledge, there is no clinically validated molecule for preventing ototoxicity, whatever the cause thereof. Accordingly, there is still a need for means to prevent ototoxicity, i.e. an agent preventing the appearance of ototoxicity or the worsening of ototoxicity.

Antifungal agents are known to treat and prevent mycoses. Some of them were previously described to be non-ototoxic (Lawrence, The Laryngoscope, 2000; Munguia & Daniel, Int J Pediatr Otorhinolaryngol., 2008), and may therefore be used for treating otomycosis (i.e. mycosis of the ear). However, no direct effect of antifungal agents on ototoxicity was previously disclosed in the art. Surprisingly, the inventors showed that the administration of antifungal agents allows preventing ototoxicity.

The present invention thus provides compounds to prevent ototoxicity.

### SUMMARY

The present invention thus relates to a compound for use for preventing ototoxicity in a subject in need thereof, wherein said compound is an antifungal agent.

In one embodiment, said compound is a compound of general formula I: 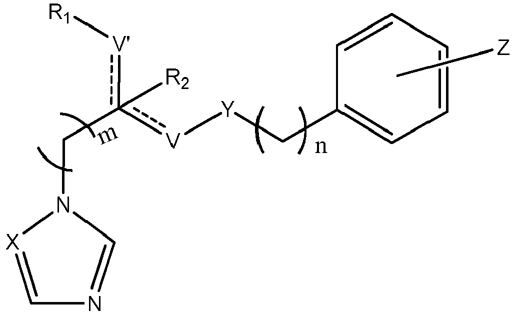 or a salt or solvate thereof, wherein
- X represents C or N atom;
- Y represents nothing, O or S atom;
- V represents nothing or an imino group;
- V' represents nothing or an imino group;
- n is 0, 1 or 2;
- m is 0, 1 or 2; and
- optionally, Z represents at least one halo, aryl, thiobenzyl or benzyl mercaptan group; preferably Z represents at least one halo group; more preferably Z represents at least one chloro or fluoro group;
- R1 or R2 may be identical or different and each is independently selected from the group of H, hydroxyl, alkyl, heteroalkyl, aryl, heteroaryl, alkylaryl, heteroalkylaryl, cycloether, oxolanyl, dioxolanyl or W1 group: 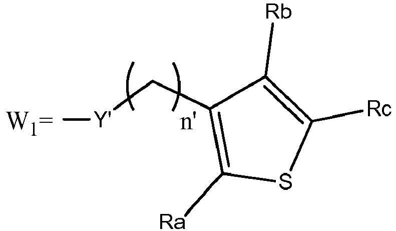
wherein,
∘ Y' represents nothing, O or S atom;
∘ n' is 0, 1 or 2;
∘ and optionally, Z' represents at least one halo, aryl or benzyl mercaptan group;
∘ Ra, Rb or Rc may be identical or different and each independently is selected from the group of H, aryl, hydroxyl, oxo, nitro, amido, carboxy, amino, cyano, alkoxy, alkyl, alkene, alkyne, cycloalkyl, cycloalkene, heteroalkyl, heteroaryl, heterocycloalkyl or halo group; preferably Ra, Rb or Rc is at least one halo group; more preferably is a chloro group;
∘ Rb and Rc may represent one fused substituent selected from the group of aryl, heteroaryl, cycloalkyl, cycloalkane or heterocycloalkyl; optionally substituted by at least one group selected from halo, aryl, hydroxyl, oxo, nitro, amido, carboxy, amino, cyano, alkoxy, alkyl, alkene, alkyne, cycloalkyl, cycloalkene, heteroalkyl or heterocycloalkyl group; preferably Rb and Rc represent an aryl group substituted by at least one chloro group;
preferably R1 or R2 may be identical or different and each is independently a benzyl, phenyl, alkyltriazolo, triazolo, thiazolo, pyrimidinyl or quinazolinone group; optionally substituted by at least one group selected from halo, aryl, hydroxyl, oxo, nitro, amido, carboxy, amino, cyano, alkoxy, alkoxyaryl, alkyl, alkene, alkyne, cycloalkyl, cycloalkene, heteroalkyl, heteroaryl, heteroalkylaryl, alkyletheraryl or heterocycloalkyl group; preferably a benzyl, phenyl, alkyltriazolo, triazolo, thiazolo, pyrimidinyl or quinazolinone group; said groups optionally substituted by at least one group selected from aryl, cycloalkyl, alkylaryl, alkylcycloalkyl, heteroaryl arylcycloalkyl, heterocycloalkyl, amidocycloalkyl or alkylamidocycloalkyl group; preferably benzyl, phenyl, alkyltriazolone, piperazinyl, arylpiperazinyl, alkylpiperazinyl, amidopiperazinyl or alkylamidopiperazinyl group; optionally substituted by alkyl, nitrile, alkyltriazolone, hydroxylalkyltriazolone, piperazinyl, amidopiperazinyl or alkylamidopiperazinyl;
R2 is a nothing when V or V' is an imido group or R1 is a cycloether, oxolanyl, dioxolanyl group;
-̅-̅-̅-̅-̅ refers to a single or a double bond,
or said compound is thiabendazole, luliconazole or omoconazole.

In one embodiment, said compound is a compound of general formula II: 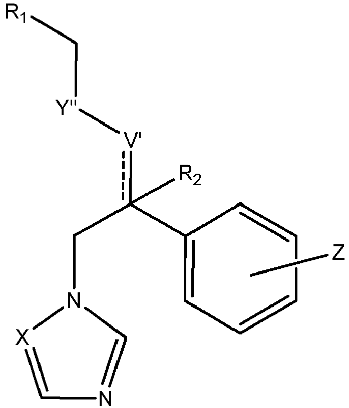 or a salt or solvate thereof, wherein
- X is C or N atom;
- V' represents nothing or an imino group;
- Y" represents nothing, O or S atom;
- R1 is selected from the aryl group; preferably a phenyl or triazolo group optionally substituted by at least one halo group, preferably by at least one chloro or fluoro group;
- R2 is H, OH or nothing when V' is an imino group;
- Z represents at least one halo group, preferably at least one chloro or fluoro group; more preferably two chloro or fluoro groups;
-̅-̅-̅-̅ refers to a single or a double bond,
preferably, the compound of formula II is selected from the list comprising miconazole, econazole, fluconazole, sulconazole and oxiconazole.

In one embodiment, said compound is a compound of general formula III: 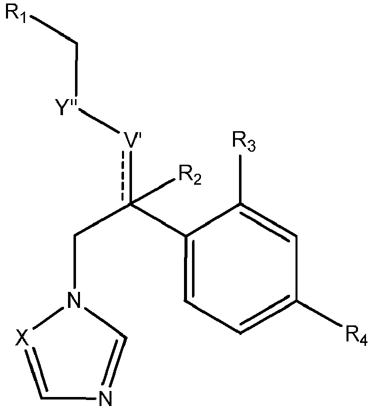 or a salt or solvate thereof, wherein
- X is C or N atom;
- V' represents nothing or an imino group;
- Y" represents nothing, O or S atom;
- R1 is selected from the aryl group; preferably a phenyl or triazolo group optionally substituted by at least one halo group, preferably by at least one chloro or fluoro group;
- R2 is H, OH or nothing when V' is an imino group;
- R3 and R4 are both chloro or fluoro group;
-̅-̅-̅-̅ refers to a single or a double bond,
preferably, the compound of formula III is selected from the list comprising miconazole, econazole, fluconazole, sulconazole and oxiconazole.

In one embodiment, said compound is a compound of general formula IV: 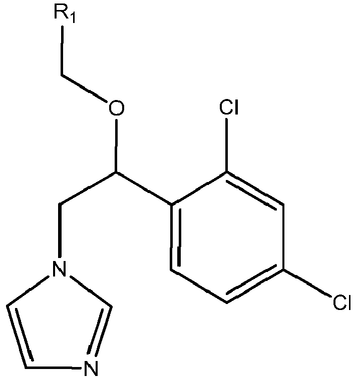 or a salt or solvate thereof,
wherein R1 is a phenyl group optionally substituted by one or more chloro group,
preferably, the compound of formula IV is selected from the list comprising miconazole and econazole.

In one embodiment, ototoxicity is induced:
- by chemotherapeutic drugs, preferably selected from vincristine or platinum based chemotherapeutic agent, more preferably cisplatin, carboplatin, oxiplatin or bis-platinate;
- by antibiotic agents, preferably selected from aminoglycoside antibiotic or macrolide antibiotic;
- by a diuretic; or
- by salicylate.

In one embodiment, said ototoxicity is cochlear toxicity or vestibular toxicity.

In one embodiment, said subject is a patient without fungal infection. In one embodiment, said subject is treated or is planned to be treated with a drug inducing ototoxicity.

The present invention also relates to a composition for use for preventing ototoxicity, said composition comprising a compound as hereinabove described. In one embodiment, the composition is in a form adapted for systemic administration.

In one embodiment, the composition comprises from about 10 mg/mL to about 10000 mg/mL of said compound.

The present invention also relates to a pharmaceutical composition for use for preventing ototoxicity, comprising a compound as hereinabove described, or a composition as hereinabove described, and at least one pharmaceutically acceptable excipient.

The present invention also relates to a medicament for use for treating or preventing ototoxicity, comprising a compound as hereinabove described, or a composition as hereinabove described.

### DEFINITIONS

In the present invention, the following terms have the following meanings:
- **"Ototoxicity"** refers to lesions to the ear induced by ototoxic agents or environmental ototoxic conditions (i.e. agents or conditions toxic to the ear), specifically lesions of the cochlea and/or of the vestibule. Examples of symptoms of ototoxicity include, but are not limited to, hearing loss, balance loss, tinnitus, vertigo and dizziness. As used herein, ototoxicity may include (1) cochlear toxicity which results in hearing loss and tinnitus and (2) vestibular toxicity which results in loss of balance. The mechanism of ototoxicity may include the lesion or destruction of neurons and/or sensory cells (hair cells) and/or endolymph producing cells of the cochlea or the vestibule. The term "neurons" refers to neural cells of the cochlea and the vestibule, which form the Vestibulocochlear (VIII)/auditory nerve. Therefore, ototoxicity include cochlear and vestibular toxicity, and thereby auditory nerve toxicity.
- "**Cochlea**" refers to the ventral region of the inner ear, containing the organ of Corti that comprises mechanosensory hair cells and supporting cells. The cochlea is dedicated to the auditory function, converting sound pressure patterns from the outer ear into electrochemical impulses which are passed on to the brain via the auditory nerve.
- "**Vestibule**" refers to the organ of the inner ear dedicated to sense of balance. Its function consists in the detection of linear and angular accelerations, with gravity as referential force, of the head in order to transmit to the brain information on movements to achieve the equilibration function in collaboration with visual and proprioceptive information. It is constituted of supporting and mechanosensory hair cells that convert mechanical force into electrical potentials.
- "**Antifungal agent**" refers to a fungicidal agent, i.e. an agent that kills or inhibits fungi or fungal spores. In one embodiment, the term "antifungal agent" refers to an agent which inhibits the enzyme lanosterol 14 α-demethylase, an enzyme necessary to convert lanosterol to ergosterol.
- "**Therapeutically effective amount**" means level or amount of compound of the invention that is aimed at, without causing significant negative or adverse side effects to the target, (1) delaying or preventing the onset of ototoxicity; or (2) slowing down or stopping the progression, aggravation, or deterioration of one or more symptoms of ototoxicity. A therapeutically effective amount may be administered prior to the onset of ototoxicity, for a prophylactic or preventive action. Alternatively or additionally, the therapeutically effective amount may be administered after initiation of ototoxicity, for preventing a worsening of ototoxicity.
- "**Dosage regimen**" refers to the schedule of doses of a therapeutic agent per unit of time, including: the time between doses or the time when the dose(s) are to be given, and the amount of a medicine to be given at each specific time.
- "**Salt**" the compounds of the invention may be in the form of a salt, preferably a pharmaceutically acceptable salt. Pharmaceutically acceptable salts include the acid addition and base salts thereof.
- "**Solvate**" is used in the present invention to describe a molecular complex comprising the compound of the invention and one or more pharmaceutically acceptable solvent molecule, for example ethanol. The term "hydrate" is employed when said solvent is water.
- "**About**" preceding a figure means plus or less 10% of the value of said figure.
- "**Alkyl**" refers to compound of formula CnH2n+1, wherein n is a number greater than or equal to 1. Generally, alkyl groups of this invention comprise from 1 to 14 carbon atoms. Alkyl groups may be linear or branched and may be substituted. Examples of alkyl groups are methyl, ethyl, n-propyl, i-propyl, butyl and its isomers (e.g. n-butyl, i-butyl and t-butyl); pentyl and its isomers, hexyl and its isomers.
- "**Alkene**" refers to any linear or branched hydrocarbon chain having at least one double bond, preferably 2 to 12 carbon atoms.
- "**Alkyne**" refers to any linear or branched hydrocarbon chain having at least one triple carbon bond; preferably 2 to 12 carbon atoms.
- "**Alkoxy**" refers to any O-alkyl or O-aryl group.
- "**Aryl**" refers to any polyunsaturated, aromatic hydrocarbyl group having a single ring (i.e. phenyl) or multiple aromatic rings fused together (e.g. naphtyl) or linked covalently, typically containing 6 to 50 atoms; preferably 6 to 10, wherein at least one ring is aromatic.
- "**Heteroaryl**" refers to aryl group having at least one atom that is not carbon or hydrogen; preferably, said atom is selected from N, S, P or O.
- "**Cycloalkyl**" refers to a cyclic alkyl group, that is to say, a monovalent, saturated, or unsaturated hydrocarbyl group having 1 or 2 cyclic structure. Cycloalkyl includes all saturated hydrocarbon groups containing 1 to 2 rings, including monocyclic or bicyclic groups. Cycloalkyl groups may comprise 3 or more carbon atoms in the ring and generally, according to this invention comprise from 3 to 10, more preferably from 3 to 8 carbon atoms still more preferably from 3 to 6 carbon atoms. The further rings of multi-ring cycloalkyls may be either fused, bridged and/or joined through one or more spiro atoms. Examples of cycloalkyl groups include but are not limited to cyclopropyl, cyclobutyl, cyclopentyl, cyclohexyl.
- "**Cycloalkene**" refers to a cyclic alkene group that is to say, a monovalent, saturated, or unsaturated hydrocarbyl group having 1 or 2 cyclic structure. Cycloalkene includes all unsaturated hydrocarbon groups containing 1 to 2 rings, including monocyclic or bicyclic groups. Cycloalkene groups may comprise 3 or more carbon atoms in the ring and generally, according to this invention comprise from 3 to 10, more preferably from 3 to 8 carbon atoms still more preferably from 3 to 6 carbon atoms. The further rings of multi-ring cycloalkene may be either fused, bridged and/or joined through one or more spiro atoms. Examples of cycloalkene groups include but are not limited to cyclopropenyl, cyclobutenyl, cyclopentenyl, cyclohexenyl.
- "**Heteroalkyl**" refers to refers to alkyl group having at least one atom that is not carbon or hydrogen; preferably, said atom is selected from N, S, P or O.
- "**Heterocycloalkyl**" refers to a cycloalkyl group having at least one atom that is not carbon or hydrogen; preferably, said atom is selected from N, S, P or O.

### DETAILED DESCRIPTION

The present invention relates to a compound for preventing, or for use in preventing, ototoxicity. Preferably, the present invention relates to a compound for preventing the appearance or the worsening of ototoxicity and/or symptoms thereof.

In one embodiment of the invention, the compound is an antifungal agent. In one embodiment, the antifungal agent is selected from the group comprising miconazole, sulconazole, econazole, ketoconazole, clotrimazole, bifonazole, butoconazole, fenticonazole, isoconazole, oxiconazole, sertaconazole, thiabendazole, tioconazole, fluconazole, itraconazole, ravuconazole, posaconazole, voriconazole, luliconazole, omoconazole, albaconazole, isavuconazole and terconazole.

In one embodiment, the antifungal agent belongs to the class of the azole antifungal drugs, preferably imidazole antifungals and triazole antifungals.

Examples of imidazole antifungals include, but are not limited to, bifonazole, butoconazole, clotrimazole, econazole, fenticonazole, isoconazole, ketoconazole, luliconazole, miconazole, omoconazole, oxiconazole, sertaconazole, sulconazole and tioconazole.

Examples of triazole antifungals include, but are not limited to, albaconazole, fluconazole, isavuconazole, itraconazole, posaconazole, ravuconazole, terconazole and voriconazole.

In one embodiment of the invention, the compound of the invention is a compound of general formula I or a salt or solvate thereof, wherein the general formula I is as follows, 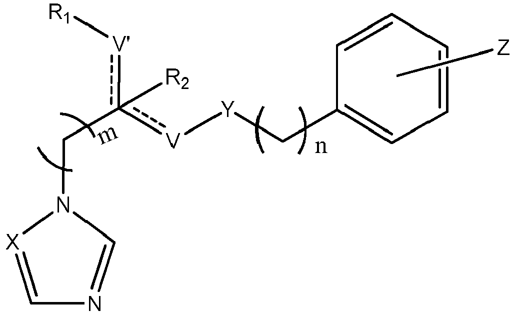 wherein
- X represents C or N atom;
- Y represents nothing, O or S atom;
- V represents nothing or an imino group;
- V' represents nothing or an imino group;
- n is 0, 1 or 2;
- m is 0, 1 or 2; and
- optionally, Z represents at least one halo, aryl, thiobenzyl or benzyl mercaptan group; preferably Z represents at least one halo group; more preferably Z represents at least one chloro or fluoro group;
- R1 or R2 may be identical or different and each is independently selected from the group of H, hydroxyl, alkyl, heteroalkyl, aryl, heteroaryl, alkylaryl, heteroalkylaryl, cycloether, oxolanyl, dioxolanyl or W1 group: 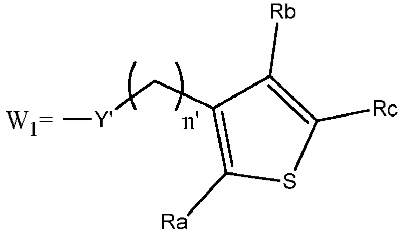 wherein,
   - Y' represents nothing, O or S atom;
   - n' is 0, 1 or 2;
   - and optionally, Z' represents at least one halo, aryl or benzyl mercaptan group;
   - Ra, Rb or Rc may be identical or different and each independently is selected from the group of H, aryl, hydroxyl, oxo, nitro, amido, carboxy, amino, cyano, alkoxy, alkyl, alkene, alkyne, cycloalkyl, cycloalkene, heteroalkyl, heteroaryl, heterocycloalkyl or halo group; preferably Ra, Rb or Rc is at least one halo group; more preferably is a chloro group;
   - Rb and Rc may represent one fused substituent selected from the group of aryl, heteroaryl, cycloalkyl, cycloalkane or heterocycloalkyl; optionally substituted by at least one group selected from halo, aryl, hydroxyl, oxo, nitro, amido, carboxy, amino, cyano, alkoxy, alkyl, alkene, alkyne, cycloalkyl, cycloalkene, heteroalkyl or heterocycloalkyl group; preferably Rb and Rc represent an aryl group substituted by at least one chloro group;
preferably R1 or R2 may be identical or different and each is independently a benzyl, phenyl, alkyltriazolo, triazolo, thiazolo, pyrimidinyl or quinazolinone group; optionally substituted by at least one group selected from halo, aryl, hydroxyl, oxo, nitro, amido, carboxy, amino, cyano, alkoxy, alkoxyaryl, alkyl, alkene, alkyne, cycloalkyl, cycloalkene, heteroalkyl, heteroaryl, heteroalkylaryl, alkyletheraryl or heterocycloalkyl group; preferably a benzyl, phenyl, alkyltriazolo, triazolo, thiazolo, pyrimidinyl or quinazolinone group; said groups optionally substituted by at least one group selected from aryl, cycloalkyl, alkylaryl, alkylcycloalkyl, heteroaryl arylcycloalkyl, heterocycloalkyl, amidocycloalkyl or alkylamidocycloalkyl group; preferably benzyl, phenyl, alkyltriazolone, piperazinyl, arylpiperazinyl, alkylpiperazinyl, amidopiperazinyl or alkylamidopiperazinyl group; optionally substituted by alkyl, nitrile, alkyltriazolone, hydroxylalkyltriazolone, piperazinyl, amidopiperazinyl or alkylamidopiperazinyl;
R2 is a nothing when V or V' is an imido group or R1 is a cycloether, oxolanyl, dioxolanyl group;
-̅-̅-̅-̅-̅ refers to a single or a double bond.

Examples of compounds of formula I include, but are not limited to, miconazole, sulconazole, econazole, ketoconazole, clotrimazole, bifonazole, butoconazole, fenticonazole, isoconazole, oxiconazole, sertaconazole, tioconazole, fluconazole, itraconazole, ravuconazole, posaconazole and voriconazole.

In one embodiment of the invention, the compound of general formula I is a compound of general formula II: 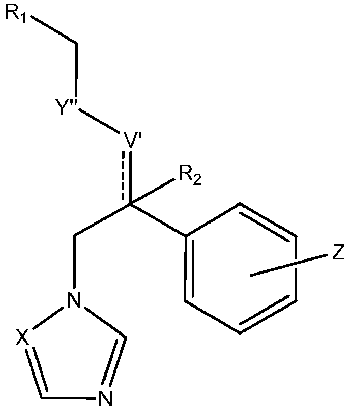 or a salt or solvate thereof,
wherein
- X is C or N atom;
- V' represents nothing or an imino group;
- Y" represents nothing, O or S atom;
- R₁ is selected from the aryl group; preferably a phenyl or triazolo group optionally substituted by at least one halo group, preferably by at least one chloro or fluoro group;
- R₂ is H, OH or nothing when V' is an imino group;
- Z represents at least one halo group, preferably at least one chloro or fluoro group;
   more preferably two chloro or fluoro groups;
-̅-̅-̅-̅-̅ refers to a single or a double bond.

Examples of compounds of formula II include, but are not limited to, fluconazole, miconazole, sulconazole, econazole, oxiconazole, isoconazole and tioconazole.

In one embodiment, the compound of general formula II is not isoconazole or tioconazole.

In one embodiment, the compound of general formula II is selected from the group comprising fluconazole, miconazole, sulconazole, econazole or oxiconazole.

In one embodiment, the compound of general formula I is a compound of general formula III: 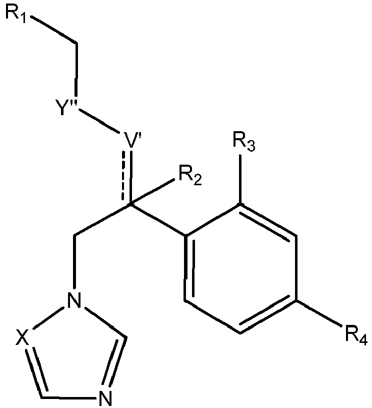 or a salt or solvate thereof, wherein
- X is C or N atom;
- V' represents nothing or an imino group;
- Y" represents nothing, O or S atom;
- R1 is selected from the aryl group; preferably a phenyl or triazolo group optionally substituted by at least one halo group, preferably by at least one chloro or fluoro group;
- R2 is H, OH or nothing when V' is an imino group;
- R3 and R4 are both chloro or fluoro group;
-̅-̅-̅-̅-̅ refers to a single or a double bond.

Examples of compounds of formula III include, but are not limited to, fluconazole, miconazole, sulconazole, econazole, oxiconazole, isoconazole and tioconazole.

In one embodiment, the compound of general formula III is not isoconazole or tioconazole.

In one embodiment, the compound of general formula III is selected from the group comprising fluconazole, miconazole, sulconazole, econazole or oxiconazole.

In a preferred embodiment, the compound of general formula I is a compound of general formula IV: 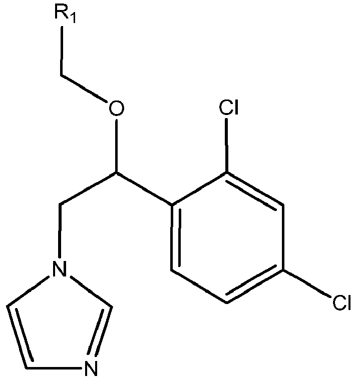 or a salts or solvate thereof,
wherein R1 is a phenyl group optionally substituted by one or more chloro group.

Examples of compounds of formula IV include, but are not limited to, miconazole, econazole and isoconazole.

In one embodiment, the compound of general formula IV is not isoconazole.

In one embodiment, the compound of general formula IV is miconazole or econazole.

In one embodiment, the compound of the invention is miconazole, econazole, fluconazole, sulconazole or oxiconazole. In one embodiment, the compound of the invention is miconazole, or econazole.

In one embodiment, the compound of the invention is selected from the group comprising thiabendazole, luliconazole and omoconazole.

In a first embodiment of the invention, ototoxicity is a drug-induced ototoxicity. Examples of drugs that may induce ototoxicity include, but are not limited to, chemotherapy agents, antibiotic agents, excitatory neurotoxins, quinine and analogs thereof, salicylate and analogs thereof, and loop-diuretics.

In one embodiment of the invention, ototoxicity is a chemotherapy-induced ototoxicity, induced for example by a platinum-based chemotherapeutic agent or vincristine. In one embodiment of the invention, ototoxicity is induced by a platinum-based chemotherapeutic agent such as cisplatin, carboplatin, oxaliplatin, satraplatin, picoplatin, nedaplatin, triplatin and lipoplatin.

In another embodiment of the invention, ototoxicity is induced by an antibiotic agent, such as an aminoglycoside antibiotic or a macrolide antibiotic. In one embodiment, ototoxicity is induced by an aminoglycoside antibiotic such as gentamicin, streptomycin, kanamycin or tobramycin.

In another embodiment of the invention, ototoxicity is induced by an excitatory neurotoxin such as glutamate.

In another embodiment of the invention, ototoxicity is induced by quinine or an analog.

In another embodiment of the invention, ototoxicity is induced by salicylate or an analog.

In another embodiment of the invention, ototoxicity is induced by loop-diuretics.

In one embodiment, ototoxicity is vestibular toxicity, i.e. toxicity inducing impairment of the vestibule function leading to vestibular deficits. In one embodiment, said impairment may be due to vestibular hair cells insults or loss. In one embodiment, said impairment may be due to vestibular neurons, preferably primary vestibular neurons, insults or loss. In another embodiment, said impairment may be due to lesion in the vestibule. In another embodiment, said impairment may be due to dark cells insults or loss, wherein dark cells are endolymph producing cells in the vestibule.

In another embodiment, ototoxicity is cochlear toxicity, i.e. toxicity inducing damages of the cochlea. In one embodiment, said impairment may be due to cochlear hair cells insults or loss. In one embodiment, said impairment may be due to cochlear neurons, preferably cochlear primary neurons, insults or loss. In another embodiment, said impairment may be due to lesion in the cochlea. In another embodiment, said impairment may be due to stria vascularis insults or loss, wherein stria vascularis is the cellular site of endolymph production in the cochlea. In another embodiment, said impairment may be due to the destruction of sensory epithelium or cochlear neurons. The present invention also relates to a composition for preventing (or for use in preventing) ototoxicity, comprising a compound of the present invention.

The present invention also relates to a pharmaceutical composition for preventing (or for use in preventing) ototoxicity, comprising a compound of the invention and at least one pharmaceutically acceptable excipients.

The term "pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The present invention also relates to a medicament for preventing (or for use in preventing) ototoxicity, comprising a compound of the invention, a composition or a pharmaceutical composition of the present invention.

Preferably, the composition, the pharmaceutical composition or the medicament of the invention comprises a therapeutically effective amount of the compound of the invention.

In one embodiment, the therapeutically effective amount ranges from about 10 to about 10000 mg/ml of the composition, pharmaceutical composition or medicament of the invention, preferably 100 to about 5000 mg/ml, more preferably from about 200 to about 2000 mg/ml of the composition, pharmaceutical composition or medicament of the invention.

In one embodiment, the therapeutically effective amount ranges from about 10 to about 10000 mg/g of the composition, pharmaceutical composition or medicament of the invention, preferably 100 to about 5000 mg/g, more preferably from about 200 to about 2000 mg/g of the composition, pharmaceutical composition or medicament of the invention.

It will be understood that the total daily usage of the compound of the invention, composition, pharmaceutical composition and medicament of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from about 10 to about 10000 mg per adult per day, preferably 100 to about 5000, more preferably from about 200 to about 2000 mg per adult per day. Preferably, the compositions contain 10, 50, 100, 250, 500, 1000 and 2,000 mg of the active ingredient for the symptomatic adjustment of the dosage to the patient to be treated. A medicament typically contains from about 10 to about 10000 mg of the active ingredient, preferably 100 to about 5000, more preferably from about 200 to about 2000 mg of the active ingredient. An effective amount of the drug is ordinarily supplied at a dosage level from 0.1 mg/kg to about 100 mg/kg of body weight per day, preferably from about 1 mg/kg to 40 mg/kg of body weight per day, more preferably from about 2 mg/kg to 20 mg/kg of body weight per day.

The compound of the invention can be administered prior to, during or after ototoxicity has been induced. In one embodiment, the compound of the invention is administered during the induction of ototoxicity, i.e. for example is co-administered with the drug inducing ototoxicity. As used herein, "prior to" refers to an administration few hours or minutes before exposition to drug inducing ototoxicity, and "after" refers to an administration few minutes or hours after.

In one embodiment, the composition, pharmaceutical composition or medicament comprises sustained-release matrices, such as biodegradable polymers.

In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the composition, pharmaceutical composition or medicament contains vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compound of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The compounds of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with one or several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 mL of isotonic NaCl solution and either added to 1000 mL of hypodermoclysis fluid or injected at the proposed site of infusion. Some variation in dosage will necessarily occur depending on the condition of the subject being treated.

The person responsible for administration will, in any event, determine the appropriate dose for the individual subject. The compounds of the invention may be formulated within a therapeutic mixture to comprise about 10 to 10000 milligrams, preferably from about 100 to 4000 milligrams, more preferably from about 200 to 2000 per dose or so. Multiple doses can also be administered.

In addition to the compounds of the invention formulated for parenteral administration, such as intravenous or intramuscular injection, other pharmaceutically acceptable forms include, e.g. tablets or other solids for oral administration; liposomal formulations; time release capsules; and any other form currently used.

In one embodiment, the compound of the invention is topically administered, preferably is topically applied on the outer ear or on the ear canal. Examples of formulations adapted to topical administration include, but are not limited to, ear drops, solutions or topical gels.

In a preferred embodiment, the compound of the invention is systemically administered, such as, for example, orally administered, intranasally administered or injected (including, for example, intraperitoneal, intravenously or intramuscularly injected).

As used herein, the term "subject" refers to a mammal, preferably a human. In one embodiment, a subject may be a "patient", i.e. a warm-blooded animal, preferably a pet (such as, for example, a cat, a dog, a ferret, a rabbit, a guinea pig or the like), more preferably a human, who/which is awaiting the receipt of, or is receiving medical care or was/is/will be the object of a medical procedure, or is monitored for the development of a disease.

In one embodiment of the invention, the subject refers to a patient without fungal infection, preferably without otomycosis.

In one embodiment of the invention, the patient has been treated, is concomitantly treated or is planned to be treated with a drug-inducing ototoxicity.

In one embodiment, symptoms of ototoxicity have been diagnosed in the patient. In another embodiment, the patient has no onset of symptoms of ototoxicity.

The present invention also relates to a method for preventing ototoxicity in a subject in need thereof comprising administering to the subject a therapeutically effective amount of a compound of the invention as described above.

In one embodiment, a composition, pharmaceutical composition or medicament of the invention is administered to the subject.

The invention will be further illustrated by the following figures and examples. However, these examples and figures should not be interpreted in any way as limiting the scope of the present invention.

### BRIEF DESCRIPTION OF THE DRAWINGS

**Figure 1** is a graph showing the *in vivo* impact of miconazole on cisplatin-induced vestibular toxicity.
**Figure 2** is a graph showing the *in vivo* impact of econazole on cisplatin-induced vestibular toxicity.
**Figure 3** is a graph showing the *in vivo* impact of oxiconazole on cisplatin-induced vestibular toxicity.
**Figure 4** is a graph showing the *in vivo* impact of fluconazole on cisplatin-induced vestibular toxicity.
**Figure 5** is a graph showing the *in vivo* impact of bifonazole on cisplatin-induced vestibular toxicity.
**Figure 6** is a graph showing the ear concentration of compound 30 minutes (A) or 180 minutes (B) after systemic administration.
**Figure 7** is a graph showing the comparison between the impact of miconazole and cimetidine on cisplatin-induced vestibular toxicity.
**Figure 8** is a graph showing the comparison between the impact of econazole and cimetidine on cisplatin-induced vestibular toxicity.
**Figure 9** is a histogram representing the number of hair cells and hair bundles per 100 µm² of utricle when cisplatin is administrated alone, with miconazole or with econazole to a dose ratio 1/1 of compound/cisplatin.
**Figure 10** is a graph showing the *in vivo* impact of miconazole or econazole on cisplatin-induced vestibular toxicity (dose ratio: 1/1).

### EXAMPLES

The present invention is further illustrated by the following examples.

### MATERIALS AND METHODS

### Transtympanic induction of cisplatin ototoxicity and pharmacological prevention

Ototoxicity is induced by a transtympanic injection (50µl) of cisplatin (2mg/ml in saline; 6.7 mM). To test the preventive effect of compounds, a determined ratio of corresponding molar dose of compounds are premixed and injected transtympanically with cisplatin. For example, for the dose ratio 1/2 of compound/cisplatin, a solution of 3.5 mM of compound of interest in addition to the 6.7 mM of cisplatin in saline is prepared and, 50 µl of this mixed saline solution is injected transtympanically at T0. Different control animals are run in parallel: saline only, cisplatin (6.7 mM) only to control the initial lesion, and the compounds of interest alone at the highest tested dose (13.4, 26.8 or 53.6 mM) in saline to control potential ototoxicity of the compound independently of cisplatin, are with cisplatin. The level of efficacy for the different dose ratio of compounds/cisplatin is compared to 8/1 dose ratio of cimetidine/cisplatin whose efficiency to prevent cisplatin induced ototoxicity is known.

### Evaluation of otototoxicity: scoring of behavioral vestibular deficit

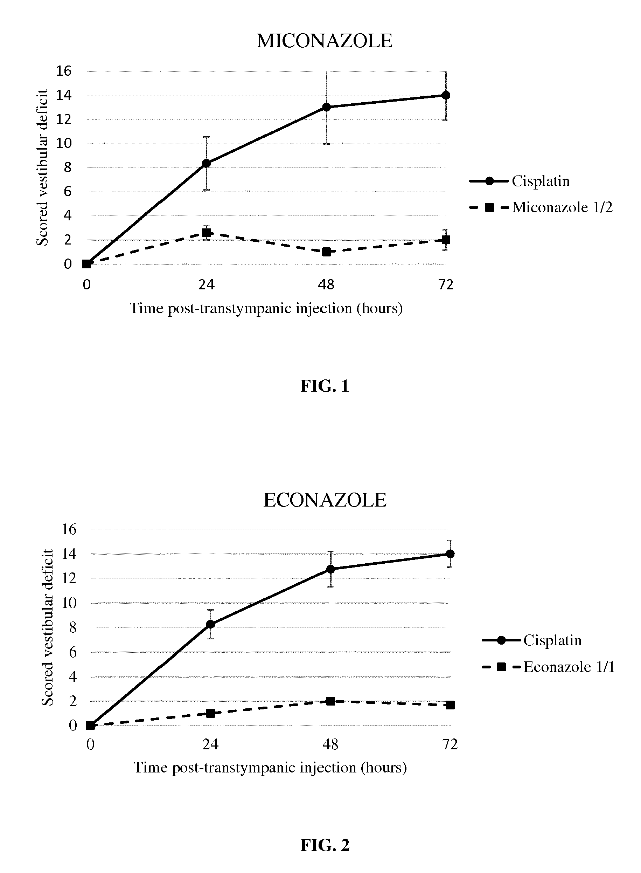

Detailed evaluation of scored vestibular deficits was previously detailed in Vignaux et al., 2012. In summary, vestibular deficit associated behaviors were scored on a scale from 0 to 4, respectively ranging from normal behavior (rating 0) to maximal identified vestibular impairment (rating 4). Seven different tests were sequentially scored and totaled to rate the vestibular deficit: 1- head bobbing (abnormal intermittent backward extension of the neck); 2- circling (stereotyped movement ranging from none to compulsive circles around the hips of the animal); 3- retro-pulsion (typical backward walk reflecting vestibular disturbance); 4- tail-hanging reflex (normally induces a forelimb extension to reach the ground, unilateral deficit results in axial rotation of the body); 5- contact-inhibition reflex (normally leads animal to release hold grip on metal grid in a supine position when their back touch the ground; in case of vestibular deficit with lack of full body orientation reference, animal retains grip on the grid in a supine position); 6- air-righting reflex (necessary for rats to land on all four feet when falling from a supine position; vestibular dysfunction impairs normal body repositioning with a maximal deficit leading the animal to land on its back when dropped from a height of 40 cm onto a foam cushion); 7-head tilt. Rats were scored for vestibular deficits just before the transtympanic injection, T0 and, evaluated at 24, 48 & 72 hours after the injection (**Figures 1 to 5**). Scores were expressed as mean (± sem). Four animals of each group were tested.

### Inner ear exposure of compounds

In clinical condition, cisplatin is administered via the intravenous route. To evaluate potential efficacy of the compounds of interest to prevent from cisplatin induced ototoxicity following chemotherapy treatment, we evaluate the capacity of each compounds to reach the inner ear compartment. A systemic administration (40 mM; intravenous) of cisplatin or the compound of interest was realized, temporal bones were excised 90 or 180 min after the systemic injection. Inner ear tissue was extracted and the quantity of compounds analyzed by LC/MS appropriate methods. Quantity are expressed as mean (± sem). Four animals of each group were sampled for each time point.

### Evaluation of ototoxicity: quantification of hair cells in vestibular epitheliums.

To evaluate hair cell loss induced by transtympanic injection of cisplatin and the potential preventive action of co-administraton of miconazole or econazole at an equimolar dose (1/1), temporal bones of rats were fixed 3 days after the transtympanic injection and after the last behavioral evaluation by immersion in PFA 4% for 1hr. Vestibular sensory epitheliums, the utricles were excised and dissected out. Labeling of hair bundles was processed using Alexa488-conjugated phalloidin (1:700) overnight and labelling of hair cells was obtained by immunolabelling of Myosin VIIa protein (1:1000) counterstained with Alexa653-conjugated secondary antibodies (1:700). After rinsing with PBS, sensory epitheliums were mounted in Moviol (Calbiochem). Fluorescent labels were observed and image digitalized using an apotome microscope (Zeiss). Hair bundles and hair cells per utricle were quantified using ImageJ software. Statistical analysis was processed with SigmaPlot software (Systat Software).

### RESULTS

### Prevention of ototoxicity induced by cisplatin

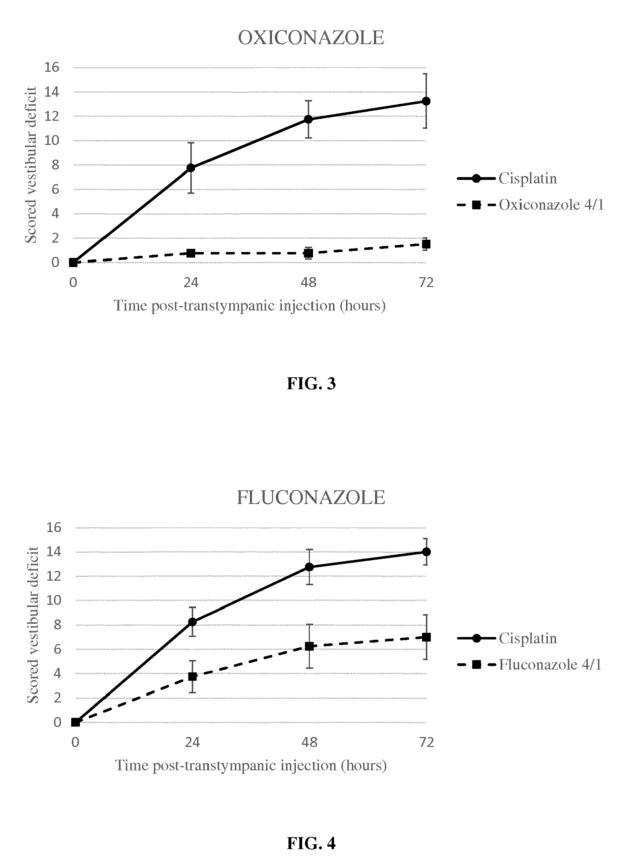

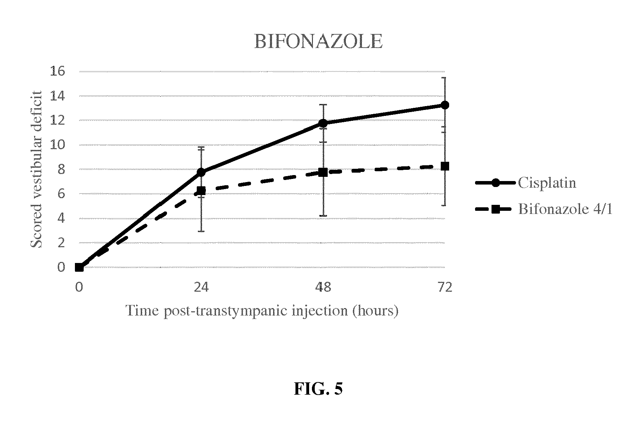

In our conditions, the vestibular deficit scored when cisplatin is co-administrated with miconazole to a dose ratio 1/2 of miconazole/cisplatin barely increased (Figure 1). 72 hours after administration, it was about 2, i.e. reduced by 7 compared to those scored with cisplatin without compound. The same results were obtained with econazole to a dose ratio 1/1 (Figure 2), and with oxiconazole to a dose ratio 4/1 (Figure 3). The administration of fluconazole to a dose ratio 4/1 also reduced the vestibular deficit by 2, from 14 to 7 (Figure 4), and the administration of bifonazole to a dose ratio 4/1 reduced the vestibular toxicity from 13 to 8 (Figure 5).

These results demonstrated that the compounds of the invention have an action against ototoxicity. Therefore, these results strongly support the use of compounds of the invention for preventing ototoxicity.

### Ear concentration of compounds after systemic administration

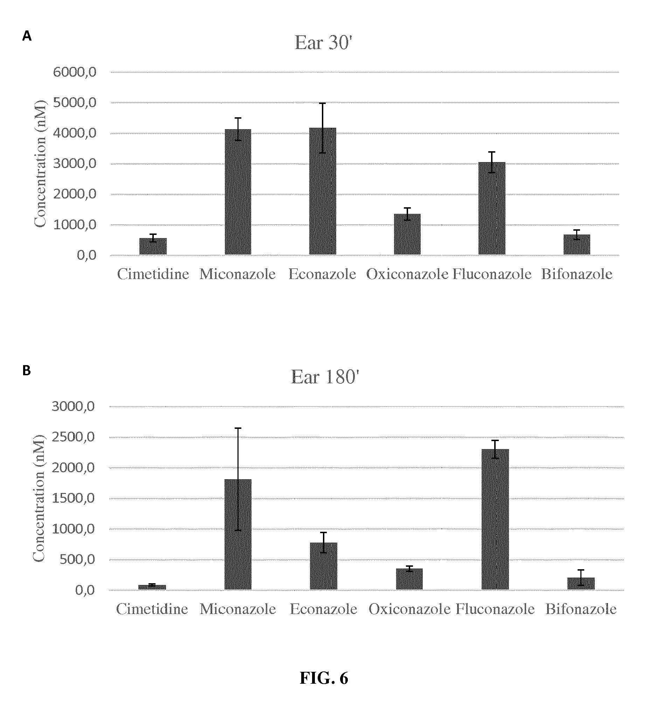

The concentration of miconazole and econazole in the ear 30 minutes after systemic administration reached more than 4000 nM (Figure 6A). In comparison, cimetidine only reached 500 nM. Oxiconazole, fluconazole and bifonazole showed higher concentration than cimetidine as well.

The concentration of compounds 3 hours after the systemic administration is still between 2,4 and 34 times higher than those of cimetidine (Figure 6B).

These results demonstrated that the systemic administration is a very effective way for compound to reach the ear. Moreover, they showed that for the same initial dose, the compounds are more concentrated is the ear than the cimetidine. Thus, a lower dose may be administered to obtain the same effective amount in the ear.

### Comparison of compounds with cimetidine

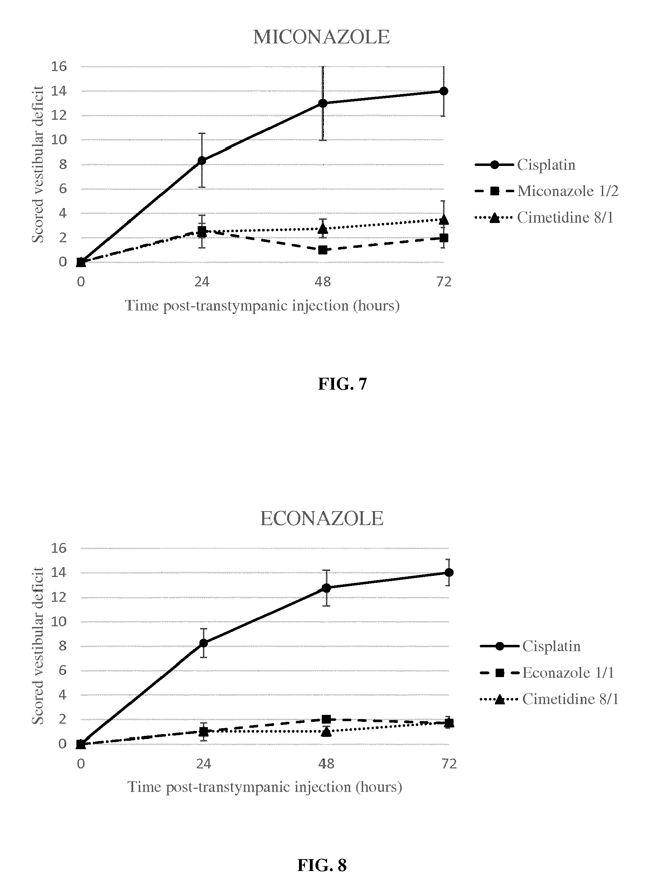

The administration of miconazole to a dose ratio 1/2 of miconazole/cisplatin showed the same efficacy of prevention of vestibular deficit than the cimetidine to a dose ratio of 8/1 (Figure 7). For econazole, a dose ratio 2/1 was sufficient to obtain the results as cimetidine 8/1 (Figure 8).

These results proved that the compounds possess a high capacity of prevention of ototoxicity. Furthermore, a significantly lower dose than those used with cimetidine is necessary to prevent ototoxicity.

Together with the previous results, the Applicant demonstrated that the compounds are very powerful agents to prevent ototoxicity and that strongly lower dose levels than cimetidine are required.

### Histological analysis

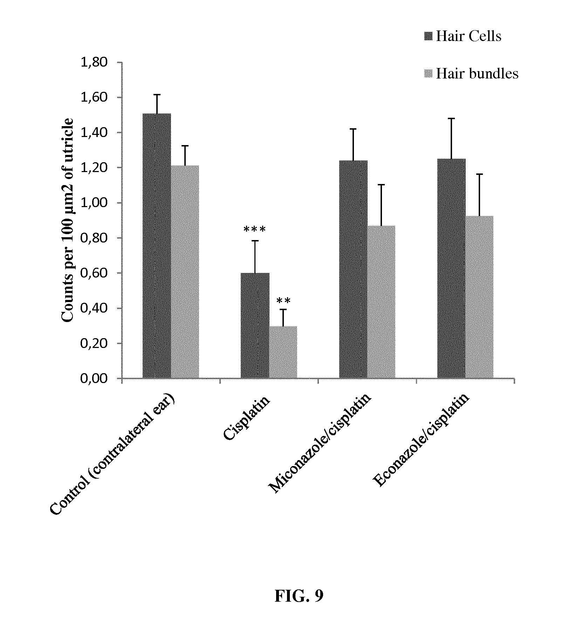

In presence of cisplatin, the number of hair cells and hair bundles strongly decreased compared to the contralateral ear used as a control, from 1.5/100 µm² to 0.6/100 µm² of utricle for hair cells and from 1.2/100 µm² to 0.5/100 µm² of utricle for hair bundles (Figure 9).

When miconazole (or econazole) was present to a dose ratio 1/1 of compound/cisplatin, hair cells only lightly decreased to from 1.5/100 µm² to 1.2/100 µm² of utricle and hair bundles to 1.2/100 µm² to 0.9/100 µm². Compared to the contralateral ear, the difference in hair cells and hair bundles quantification in presence of miconazole or econazole at a dose ratio 1/1 is not significant.

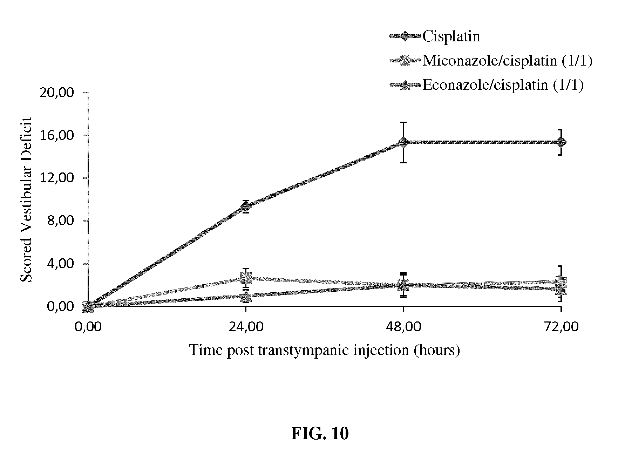

These results correlate with the behavioral analysis, which proved that the compounds are able to prevent the appearance of lesions to the ear induced by an ototoxic agent, and at a low dose level 1/1 (Figure 10).

## Claims

1. A compound for use for preventing ototoxicity in a subject in need thereof, wherein said compound is an antifungal agent.

2. The compound for use for preventing ototoxicity according to claim 1, wherein said compound is a compound of general formula I: 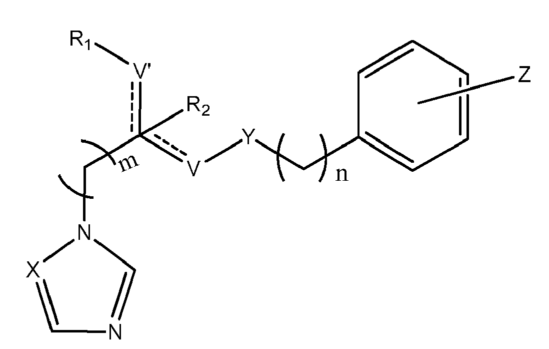 or a salt or solvate thereof,
wherein
- X represents C or N atom;
- Y represents nothing, O or S atom;
- V represents nothing or an imino group;
- V' represents nothing or an imino group;
- n is 0, 1 or 2;
- m is 0, 1 or 2; and
- optionally, Z represents at least one halo, aryl, thiobenzyl or benzyl mercaptan group; preferably Z represents at least one halo group; more preferably Z represents at least one chloro or fluoro group;
- R1 or R2 may be identical or different and each is independently selected from the group of H, hydroxyl, alkyl, heteroalkyl, aryl, heteroaryl, alkylaryl, heteroalkylaryl, cycloether, oxolanyl, dioxolanyl or W1 group: 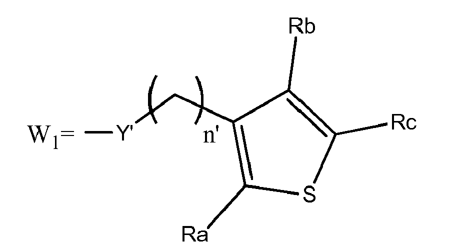 wherein,
∘ Y' represents nothing, O or S atom;
∘ n' is 0, 1 or 2;
∘ and optionally, Z' represents at least one halo, aryl or benzyl mercaptan group;
∘ Ra, Rb or Rc may be identical or different and each independently is selected from the group of H, aryl, hydroxyl, oxo, nitro, amido, carboxy, amino, cyano, alkoxy, alkyl, alkene, alkyne, cycloalkyl, cycloalkene, heteroalkyl, heteroaryl, heterocycloalkyl or halo group; preferably Ra, Rb or Rc is at least one halo group; more preferably is a chloro group;
∘ Rb and Rc may represent one fused substituent selected from the group of aryl, heteroaryl, cycloalkyl, cycloalkane or heterocycloalkyl; optionally substituted by at least one group selected from halo, aryl, hydroxyl, oxo, nitro, amido, carboxy, amino, cyano, alkoxy, alkyl, alkene, alkyne, cycloalkyl, cycloalkene, heteroalkyl or heterocycloalkyl group; preferably Rb and Rc represent an aryl group substituted by at least one chloro group;
preferably R1 or R2 may be identical or different and each is independently a benzyl, phenyl, alkyltriazolo, triazolo, thiazolo, pyrimidinyl or quinazolinone group; optionally substituted by at least one group selected from halo, aryl, hydroxyl, oxo, nitro, amido, carboxy, amino, cyano, alkoxy, alkoxyaryl, alkyl, alkene, alkyne, cycloalkyl, cycloalkene, heteroalkyl, heteroaryl, heteroalkylaryl, alkyletheraryl or heterocycloalkyl group; preferably a benzyl, phenyl, alkyltriazolo, triazolo, thiazolo, pyrimidinyl or quinazolinone group; said groups optionally substituted by at least one group selected from aryl, cycloalkyl, alkylaryl, alkylcycloalkyl, heteroaryl arylcycloalkyl, heterocycloalkyl, amidocycloalkyl or alkylamidocycloalkyl group; preferably benzyl, phenyl, alkyltriazolone, piperazinyl, arylpiperazinyl, alkylpiperazinyl, amidopiperazinyl or alkylamidopiperazinyl group; optionally substituted by alkyl, nitrile, alkyltriazolone, hydroxylalkyltriazolone, piperazinyl, amidopiperazinyl or alkylamidopiperazinyl;
R2 is a nothing when V or V' is an imido group or R1 is a cycloether, oxolanyl, dioxolanyl group;
-̅-̅-̅-̅-̅ refers to a single or a double bond,
or said compound is thiabendazole, luliconazole or omoconazole.

3. The compound for use for preventing ototoxicity according to anyone of claims 1 to 2, wherein said compound is a compound of general formula II: 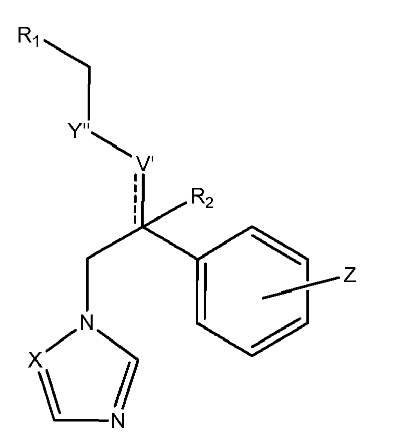 or a salt or solvate thereof,
wherein
- X is C or N atom;
- V' represents nothing or an imino group;
- Y" represents nothing, O or S atom;
- R1 is selected from the aryl group; preferably a phenyl or triazolo group optionally substituted by at least one halo group, preferably by at least one chloro or fluoro group;
- R2 is H, OH or nothing when V' is an imino group;
- Z represents at least one halo group, preferably at least one chloro or fluoro group; more preferably two chloro or fluoro groups;
-̅-̅-̅-̅ refers to a single or a double bond,
preferably, the compound of formula II is selected from the list comprising miconazole, econazole, fluconazole, sulconazole and oxiconazole.

4. The compound for use for preventing ototoxicity according to anyone of claims 1 to 3, wherein said compound is a compound of general formula III: 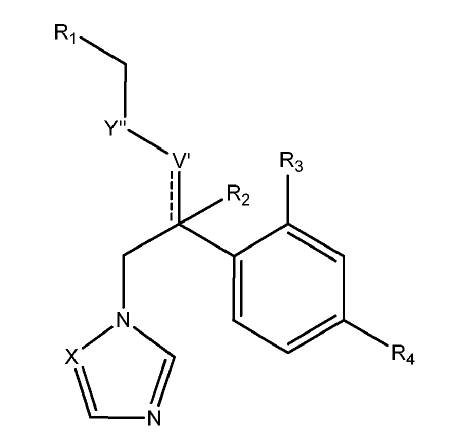 or a salt or solvate thereof,
wherein
- X is C or N atom;
- V' represents nothing or an imino group;
- Y" represents nothing, O or S atom;
- R1 is selected from the aryl group; preferably a phenyl or triazolo group optionally substituted by at least one halo group, preferably by at least one chloro or fluoro group;
- R2 is H, OH or nothing when V' is an imino group;
- R3 and R4 are both chloro or fluoro group;
-̅-̅-̅-̅ refers to a single or a double bond,
preferably, the compound of formula III is selected from the list comprising miconazole, econazole, fluconazole, sulconazole and oxiconazole.

5. The compound for use for preventing ototoxicity according to anyone of claims 1 to 4, wherein said compound is a compound of general formula IV: 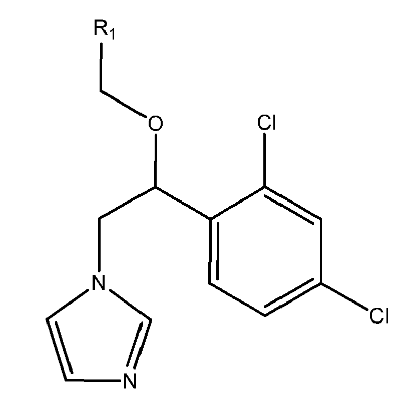 or a salt or solvate thereof,
wherein R1 is a phenyl group optionally substituted by one or more chloro group,
preferably, the compound of formula IV is selected from the list comprising miconazole and econazole.

6. The compound for use for preventing ototoxicity according to anyone of claims 1 to 5, wherein said ototoxicity is induced:
- by chemotherapeutic drugs, preferably selected from vincristine or platinum based chemotherapeutic agent, more preferably cisplatin, carboplatin, oxaliplatin or bis-platinate;
- by antibiotic agents, preferably selected from aminoglycoside antibiotic or macrolide antibiotic;
- by a diuretic; or
- by salicylate.

7. The compound for use for preventing ototoxicity according to anyone of claims 1 to 6, wherein said ototoxicity is cochlear toxicity or vestibular toxicity.

8. The compound for use for preventing ototoxicity in a subject in need thereof according to anyone of claims **1** to **7**, wherein said subject is a patient without fungal infection.

9. The compound for use for preventing ototoxicity on a subject in need thereof according to anyone of claims **1** to **8**, wherein said subject is treated or is planned to be treated with a drug inducing ototoxicity.

10. A composition for use for preventing ototoxicity, said composition comprising a compound according to anyone of claims **1** to **9**.

11. The composition for use for preventing ototoxicity according to claim **10**, wherein the composition is in a form adapted for systemic administration.

12. The composition for use for preventing ototoxicity according to claim **10** or **11**, wherein the composition comprises from about 10 mg/mL to about 10000 mg/mL of said compound.

13. A pharmaceutical composition for use for preventing ototoxicity, comprising a compound according to anyone of claims **1** to **9**, or a composition according to anyone of claims **10** to **12**, and at least one pharmaceutically acceptable excipient.

14. A medicament for use for treating or preventing ototoxicity, comprising a compound according to anyone of claims **1** to **9**, or a composition according to anyone of claims **10** to **12**.
